# EUROPEAN PATENT APPLICATION

(11) **EP 1 166 787 A2**
(43) Date of publication of application: **02.01.2002**
(21) Application number: 01600017.6
(22) Date of filing: 14.06.2001
(51) Int. Cl.: A61K 33/10, A61K 31/194, A61K 31/198, A61K 31/405

(54) **Stable amino acid based bicarbonate solutions for peritoneal dialysis and hemodialysis**

(30) Priority: 28.06.2000 GR 2000100214
(71) Applicant: Yatzidis, Hippocrates, 115 28 Athens (GR)
(72) Inventor: Yatzidis, Hippocrates, 115 28 Athens (GR)

(57) **Abstract**

The invention relates to two single and stable solutions for peritoneal dialysis (PD) and hemodialysis (HD). The solutions contain the needed electrolytes, the bicarbonate and at very small concentration (1.5mM/L) disodium hydrogen citrate. Besides, the PD solution contains the following ten indispensable amino acids, L-histidine, L-leucine, L-lysine, L-methionine, L-phenylalanine, L-threonine, L-tryptophane, L-valine, L-arginine and L-glutamine, while the HD solution contains the two dispensable amino acids, L-aspartic acid and L-glutamic acid.

## Description

### Background of The Invention

Peritoneal dialysis (PD) and hemodialysis (HD) are commonly used methods in the treatment of patient with end-stage renal disease. The uremic patient's blood, is brought into contact with dialysis solutions across either the peritoneal or an artificial membrane and is purified from the waste products based on the principles of dialysis.

Such solutions should have an electrolyte formula resembling as closely as possible that of normal plasma and contain, for exemple, sodium, potassium, chloride, calcium and magnesium ions. In PD solutions glucose is used as an osmotic agent while lactate, acetate and bicarbonate ions as buffering agents. All currently used PD solutions still have many drawbacks with serious consequences, arizing mainly from lactate, the low pH (5.2) and the high glucose concentrations and in HD solution from acetate.

At present three PD solutions containing glucose concentrations 15,25 and 42.5 g/L are used. Because of their high glucose concentrations, patients on continuous ambulatory peritoneal dialysis (CAPD) absorb daily about 200 g of glucose. This superabundant amount of glucose leads to hyperglycemia, hyperinsulinemia, hyperlipidemia and obesity, which are important risk factors for accelerating atheromatosis and arteriosclerosis. In addition, as these peritoneal solutions must be sterilized by autoclaving at a pH around 5.2 in order to avoid the carmelisation, toxic substances are generated such as glucose byproducts (5 hydroxyl methyl furfurol, furandic carboxylic acid, acetyl acrylic acid), advanced glycosylation end byproducts (AGEs), and reactive oxygen species (ROS) which damage peritoneum and its function.

On the other hand, acetate in HD solution (dialysate) is unable to control metabolic acidosis and furtermore has the potential of causing bicarbonate

On the other hand, acetate in HD solution (dialysate) is unable to control metabolic acidosis and furtermore has the potential of causing bicarbonate deficit, accumulation of acetate and its metabolites, as well as for consequent acid-base and hemodynamic disturbances. Bicarbonate has many advantages as it is the major natural buffer, with the better interdialytic hemodynamic stability and tolerance, can restore effectively acidosis with beneficial effects on protein metabolism, nutrition, the skeleton and the overall well-being of patients. However, there is a serious problem, as bicarbonate could react with calcium and magnesium and form insoluble neutral calcium and magnesium carbonate salts.

In order to overcome this drawback one approach uses two different solutions, one alkaline, containing bicarbonate, and the other acidic, containing calcium and mangesium, which are mixed on-line during dialytic therapy. A similar technique uses dry bicarbonate in a cartridge belt. It has been proposed, but not strongly supported, that in the on - line mixed solution, which ordinary has a pH of 6.8-6.9, insoluble neutral calcium and magnesium carbonate are formed late during infusion. This is correct, however, only when the solution is stored in the laboratory, including into air tight bottles, but not during treatment. In fact, studying this on-line solution at a dialyzer exit, after its contact with patient's blood through the artificial membrane, we noted that its pH reached 7.15-7.20, and it is deteriorated rapidly. More problematic is the use of on-line technique in CAPD. In fact, this is very complicated, tedious and risky as it necessitates 4 exchanges of sterile peritoneal solutions each day which are performed by the patient.

The on-line technique produces a non authentic bicarbonate solution as it becomes unstable during infusion and may decrease bicarbonate and favor the accumulation in the patient's plasma of carbonic acid, carbon dioxide, soluble hydrogen calcium and magnesium carbonates and finally insoluble neutral calcium and magnesium carbonate, the deposition of which, become evident on the wall of the connecting tubes (1-6).

From many years we have worked on the preparation of single bicarbonate solutions for PD and HD. Such imrpoved single bicarbonate solutions have been produced by rendering them true and efficient buffers with pH 7.35 by adding a quantity of glycylglycine. At this constant pH the formation and precipitation of insoluble neutral calcium and magnesium carbonate are suspended. Bicarbonate solutions were patented internationally by the author and Pierre Fabre Medicament Society (Paris, France). Numerous experimental and clinical trials have demonstrated their superiority, compared to similar standard lactate and on-line produced bicarbonate solutions ( 7-11). The high cost of glycylglycine and the need for sterilization by filtration, not accepted in many countries have made their production unacceptable.

To overcome this hindrance we have replaced successufully glycylglycine by the L-aspartic and L-glutamic acids at very low concentrations and prepared single and stable bicarbonate solutions, which are already patented in Greece and European Union (12).

We describe here the production of a single amino acid-based bicarbonate PD solution and a single bicarbonate HD solution by using simple, competent and safe technical approach.

### Description of the solutions

The particular features of single bicarbonate solutions for both PD and HD are the inclusion of disodium hydrogen citrate into their formulation, plus the inclusion of amino acids into the PD solution.

Studies in our laboratories proved that the inclusion of disodium hydrogen citrate at a very low concentration (1.5 mM/L) into the PD and HD solutions of a pH 7.25 -7.45, completely abolishes the well known reaction between bicarbonate and calcium-magnesium. As citrate disposes a potent chelating capability on divalent cations, calcium and magnesium form with citrate water soluble complexes. Consequently, after the entry of these complexes in the circulation, citrate is rapidly metabolized to bicarbonate in the liver and the released calcium and magnesium regain their pure forms. We used disodium hydrogen citrate because may control the indicated pH and also because it is more stable than trisodium citrate. Amino acids added in PD solution instead of glucose.

### Single amino acid- based bicarbonate PD solution

The exact formulation of single amino acid-based bicarbonate PD solution is listed in Table 1.

In 1968 Dudrick et al (13) introduced amino acids in parenteral i.v. nutrition and the same year Gjessing (14) added amino acids to the peritoneal solution, in an attempt to compensate for losses occuring during dialysis. In 1979 Oreopoulos et al used, for the first time. amino acids as osmotic agent in PD solution, at a concentration of 1 and 2%. instead of 1.5 and 4.25% glucose (15). Since then, handreds of relative articles have been published.

**Table 1.**

| A single and stable improved bicarbonate amino acid solution for peritoneal dialysis | | | | |
|---|---|---|---|---|
| Amino acids | | mw | mM/L | g/L |
| L-Histidine | C₆H₉N₃O₂ | 155.16 | 6 | 0.931 |
| L-Leucine | C₆H₁₃NO₂ | 131.17 | 16 | 2.098 |
| L-Lysine·HCl | C₆H₁₄N₂O₂·HCl | 182.60 | 10 | 1.826 |
| L-Methionine | C₅H₁₁NO₂S | 149.21 | 8 | 1.193 |
| L-Phenylalanine | C₉H₁₁NO₂ | 165.19 | 3 | 0.495 |
| L-Threonine | C₄H₉NO₃ | 119.12 | 13 | 1.548 |
| L-Tryptophan | C₁₁H₁₂N₂O₂ | 204.22 | 2 | 0.408 |
| L-Valine | C₅H₁₁N0₂ | 117.15 - | 18 | 2.108 |
| L-arginine | C₆H₁₄N₄O₂ | 174.20 | 3 | 0.523 |
| L-Glutamine | C₅H₁₀N₂O₃ | 146.15 | 15 | 2.192 |
| Number of amino acids : 10 | | 1544.17 | 94 | 13.322 |
| Average mw : | | 154.42 | | |

| Electrolytes | | | | g/L |
|---|---|---|---|---|
| Sodium chloride | NaCI | 58.44 | | 5.569 |
| Sodium bicarbonate | NaHCO₃ | 84.01 | | 2.940 |
| di-sodium citrate | C₆H₆Na₂O₇ 1½H₂O | 236.09 | | 0.354 |
| Calcium chloride | CaCl₂·2H₂O | 146.98 | | 0.221 |
| Magnesium chloride | MgCl₂·6H₂O | 203.21 | | 0.102 |
| Na 135+ Cl 101.5+ HCO₃ 35+ | | | | |
| Cit 1.5 + Ca 1.5+Mg 0.5 mM/L : | | | 278 | |
| pH : 7.25-7.45 Collective osmolarity :369 mOsm | | | | |

Amino acids are the structural units from which proteins are synthesized. They are unevenly distributed in the media of the body and are chemically reactive. Because amino acids possess a basic amino group they have the power to combine with either alkali or acid, acting as buffers. Amino acids can be classified, according to their chemical structures and reaction into 3 groups, in basic amino acids in which the ratio between basic amino group and acid carboxyl group is 2:1, in neutral amino acids in which the ratio is 1:1 and in acidic amino acids in which this ratio is 1:2. Dietary amino acids are a good source of energy in some organs. Thus almost 50% of oxygen consumed by the liver is used for amino acid oxidation, and in small intestine is almost 30%. In the muscle and kidney, however these figures are 7.5% and 12.5%, respectively. Not all amino acids or proteins are equal nutritionaly. Negative nitrogen balance may occasionally have to be corrected by hyperalimentation or total parenteral nutrition (TPN) amino acids, plus a source of calories in the form of fat and carboxydrate and also contain all other nutritional factors required for life such as vitamines, and minerals. Early was discovered by Osborne and Mendel (16), that the elimination of certain amino acids from the diets prevented survival or growth, while the omission of others had no deleterious effect upon the body function. This had led to the classification of amino acids as essential or nonessential (17,18) These terms are less than precise. Today, it is prefered to use the terms "indispensable"and "dispensable" amino acids. In 1987, Laidlaw and Kopple (19) in a study in healthy adult human identifield nine amino acids (isoleucine, leucine, lysine, methionine, phenylalanine, threonine, tryptophan, valine and histidine) which were shown, by classical nitrogen balance studies, to be indispensable, while the others were dispensable.

Studying our formulation of single PD solution one can note that the amino acids used are different, one to another in respect of quantitative and qualitative structure. In fact, it contains at relatively high concentration ten indispensable amino acids, histidine, leucine, lysine · HCl, methionine, phenylalamine, threonine, tryptophan, valine, arginine, and glutamine. The total amount of amino acids in our solution is approximately 13.322 g/L and its their average mw is about 154.42 with a constant pH 7.25 to 7.45 during its storage and treatment.

We consider useful to refer here briefly on the role of the numerous dispensable amino acids not included in our PD solution and of the ten indispensable amino acids, selected to be included in the solution indispensable amino acids.

Glycine, serine, proline, tyrosine, citrulline, ornitine, alanine, aspartic acid, glutamic acid and others dispensable amino acids, were rejected from our single bicarbonate PD solution. The nutritional property of these amino acids is rather poor while they dont have proved specific beneficial properties, except for an interesting cytoprotective action of glycine (6) and the antioxidant property of aspartic and glutamic acids (12). Moreover, serine is not safe, causing rats to die with anorexia, albuminuria, circulatory failure, congestion of liver and lungs (21). It is also doubtful if most of the above amino acids could form glycogen in excess and have an antiketogenic effect. In fact, only alanine, a dispensable amino acid forms glycogen and is antiketogenic, but today glutamine an indispensable now amino acid, is more potent and effective than alanine. It considerably enhances carbohydrate metabolism in all human organs with many other benificial properties (see below).

The ten amino acids included in our single bicarbonate PD solution are all indispensable which means that they cannot be synthesized in the human organism. Consequently, they may play a potent nutritional role by maintaining energy balance and thus combat and control the malnutrition of chronically uremic patients.

Histidine was disputed for many years to be an indispensable amino acid. Today, according to many studies, histidine is an indispensable amino acid (22,23). Kopple and Swendseid (24,25) established that histidine might be indispensable in chronically uremic patients, while others confirmed its indispensability (26,27). Newer metabolic studies suggest that histidine at high concentration, is associated with anorexia in animals, which could lead to protein-energy malnutrition. Histidine, the biosynthetic precursor of the neurotransmiter histamine was found in these animals to be elevated in plasma and brain five times above normal. In children, protein-energy malnutrition consistently produces symptoms of depressed food intake, edema, growth failure and psychomotor changes (28), and it must be avoided. On the contrary, in adults at a concentration up to 6 mM/L (as in our solution), histidine enhances nutrition, controls lipid metabolism (26) and maintains hemoglobin levels (29).

Leucine, lysine, methionine, threonine and valine are all well known indispensable amino acids. They are also the basic potent nutrients various studies showed that the concentrations of these amino acids could be increased manyfold without apparent toxic effects (30-34).

This is also the case with phenylalanine, and thyprophan. Phenylalanine and tryptophan are indispensable amino acids. Tryptophan is distinguished from other amino acids by its indole ring and it is the first of the amino acids to have been proved indispensable. Both are potent nutrients. They must be used at low concentrations, up to 2-3 mM/L, as they may cause transient brain disturbances in higher concentrations (35-37).

The remaining two amino acids, arginine and glutamine, merit more attention, because their roles were extended recently in many fields. Firstly, both amino acids are now classified definitely as indispensable and from this point of view they should have potent nutritional effects (38,39).

Arginine is the precursor of nitric oxide (NO) (38,39). Its supplementation in the hypercholesterolemic rabbits improves endothelium -dependent vasorelaxation, and shows that this improvement is associated with a reduction in atherogenesis (40-45). Also, in hypercholesterolemic patients arginine corrects the endothelial dysfunction in the vessels of microcirculation (46-49). Of great importance is also the effect of arginine in renal ischaemia. Schamm et al (50) demonstrated that arginine infusion was beneficial after 40 min clamping of the renal arteries, suggesting a regulatory role for the NO pathway in ischemic acute renal failure (ARF). Others shown important protective effect in the renal responce to radiocontrast material (51). Also, arginine administration in hypercholesterolemic rats, subjected to high radiocontrast nephrotoxicity, showd important and rapid attenuation (52). However, Tomé et al (39) (and others mentioned in this reference) reported recently that NO in excess, generated by irrational administration of arginine, may be directly toxic on cells in ARF and /or by its interaction with superoxide, leading to the oxidant peroxynitrite. The authors substuntiated the presence of NO synthetase (NOS) activity in the renal proximal tubules. As already mentioned arginine is the exclusive percursor of NO, a substance with a half-life of only a few seconds, and of a biological activity of 1-2 min. This activity ranges from disposal of protein metabolic waste, muscle metabolism, vascular tone regulation, immune system function, RNA synthesis, and on the release of numerous hormones (catecholamines, glucocorticoids, glucagon, growth hormone, insulin, prolactin, somatostatins), using different pathways, the disturbunce of which may produce detrimental effects. Dietary intervention with arginine may imrpove certain pathological states, such as diabetic nephropathy, cyclosporin A toxicity, salt-sensitive hypertention, ureteral obstruction, kidney hypertrophy due to high-protein feeding, and glomerular thrombosis due to administration of lipopolysachacharides (38,39,53). In order to reduce or even eliminate the detrimental effects and to take advantage of its benificial effect, we considered necessary, to lower arginine concentration around 3 mM/L in the single bicarbonate PD solution

Glutamine is the most abundant free amino acid in plasma and in intracellular pools in all tissues. In humans, glutamine accounts for approximately 20% of all amino acids. Glutamine for many years was neglected but now merits our attention, because during the last decade it was the topic of authentic skilful researchs by using isotopic techniques in human providing important and useful information. Today glutamine to be reported as the most important indispensable amino acid (54-57). A brief relation of their properties is needed. Glutamine is the precursor of proteins and nucleotides; it is the substrate and the stimulator of glucogen and gluconeogenesis in all organs and tissues, while alanine gluconeogenesis is poor and takes place in liver only; it is also the regulator of carboxydrate metabolism and antiketoic; the stimulator for the formation of lipids; glutamine suppresses the proteolysis in liver and in skeletic muscles, stimulats their proteins synthesis and it is a strong nutrient (58-71). Finally, glutamine, as also aspartic acid and glutamic acid, according our studies, is a potent scavenger of ROS (see page 13).

All components of amino acid-based bicarbonate PD solution are absolutely safe at the indicated concentrations. Dissodium hydrogen citrate concentration is trivial and after its entry in circulation, citrate is rapidly metabolized to bicarbonate (72), while, the released calcium and magnesium regain their pure forms. The consumption of disodium hydrogen citrate is trivial (0.708 g) for a 6-hr echange without any alteration of blood coagulation.

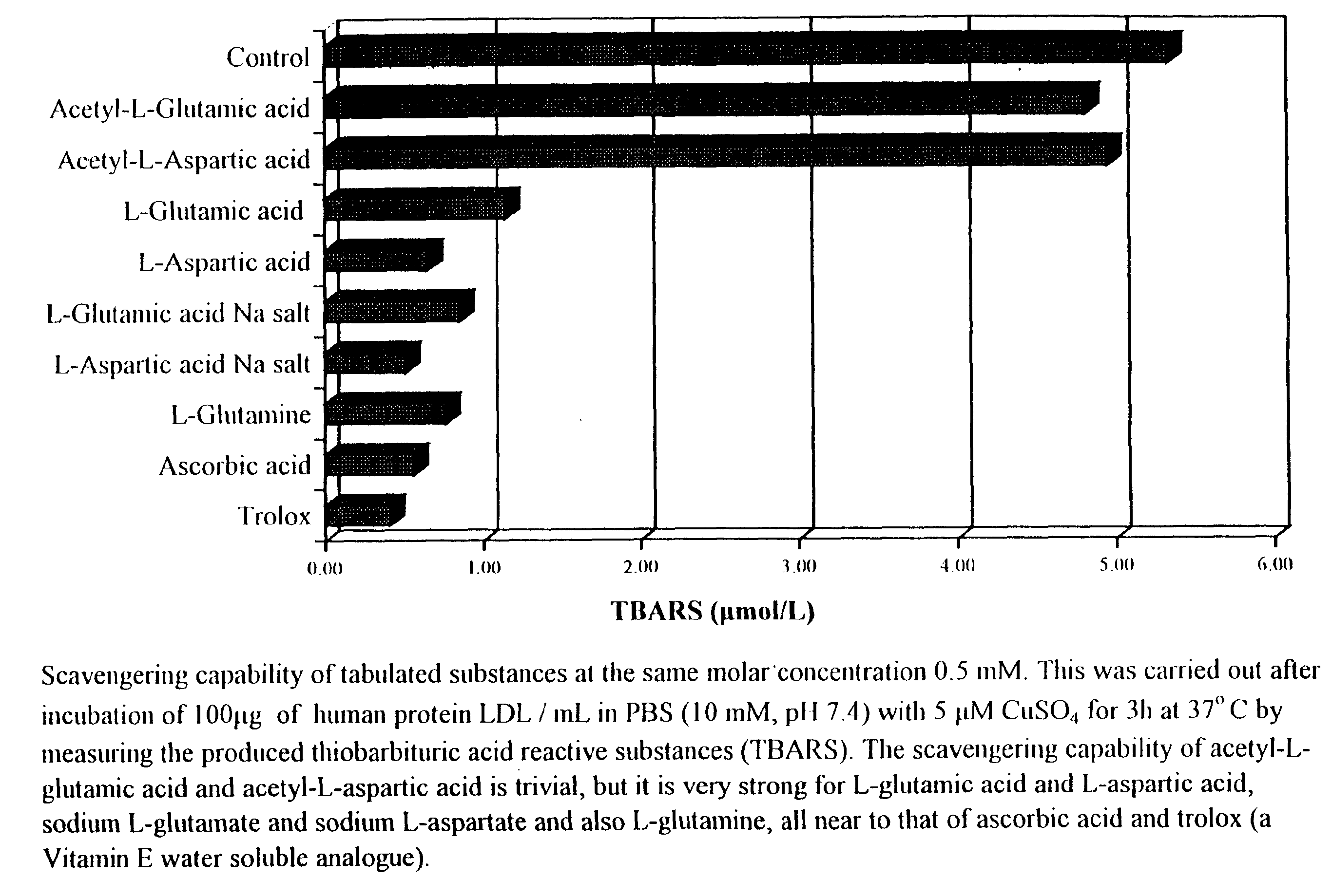

### Single bicarbonate HD solution

The exact formolution of single bicarbonate solution is listed in Table 2 (page 15).

Beside of the presence into the single bicarbonate HD solution (dialysate) of disodium hydrogen citrate at very low concentration (1.5 mM/L), it contains the required electrolytes and the two dispensable amino acids aspartic acid and glutamic acid at a very low concentration each (0.7 mM/L). These along with bicarbonate render the HD dialysate a potent buffer with a constant pH (7.25-7.45). Moreover, these amino acids, according our previous studies, are potent scavengers (see page 13), endow the HD dialysate with strong antioxidative capability, which may combat effectively ROS. As stated above the concentration of disodium hydrogen citrate as well as that of aspartic acid and glutamic acid are unimportant. Nevertheless, one could argue that, because the volume of single bicarbonate HD dialysate during a HD session is around 100 L, the total amount of disodium hydrogen citrate 35.41 g, aspartic acid 9.33 g and glutamic acid 10.3 g that comes in contact with the artificial membrane is too high. Therefore the question arises if these chemical components could by shown to be dangerous for patient. Taking into account that the HD dialysate exiting from the dialyser retains over 50% of the initial content i.e. half of the above value and that HD is used three times per week, the entry of disodium hydrogen citrate, aspartic acid and glutamic acid could not exceed 7, 2.5 and 2.75 g respectively each day. Citrate even in large amounts is rapidly metabolized to bicarbonate (see above) and the amount of both amino acids is smaller than that of daily intake with food (12).

**Table 2.**

| A single and stable improved bicarbonate solution for hemodialysis | | |
|---|---|---|
| Components | | g / 100 L |
| Sodium chloride | NaCl | 613.62 |
| Sodium bicarbonate | NaHCO₃ | 268.80 |
| Potassium chloride | KCl | 11.18 |
| di-Sodium citrate | C₆H₆Na₂O₇·1½H₂O | 35.41 |
| Calcium chloride | CaCl₂·2H₂O | 25.70 |
| Magnesium chlroride | MgCl₂·6H₂O | 10.16 |
| L-Aspartic acid | C₄H₇NO₄ | 9.33 |
| L-Glutamic acid | C₅H₉NO₄ | 10.30 |
| | | 984.50 |
| Na 140 + Cl 111 + HCO₃32 + | | |
| K 1.5 + Cit 1.5 + Ca 1.75 + | | |
| Mg 0.5 + ASP 0.7 + GLU 0.7 mM/L: 289.65 | | |
| pH : 7.25 - 7.45 Osmolarity: 287 mOsm | | |

### Preparation, sterilisation and stability

Preparation of both PD and HD solutions was carried out by dissolving chemicals in freshly produced ultrapure water taken by a procedure combining carbon perfusion, reverse osmosis and membrane filtration for retaining bacterials. For preparation of HD solution a hundredfold dry mixture of all needed chemicals (984.5 g, see Table 2) was packed and stored in an air tight plastic bottle at room temperature. Thus may serve for preparing adequate dialysate for a routine HD session, by dissolving its content at 100 liters of freshly ultrapure water made by hand or mechanically.

PD solution could be sterilized by filtration, and also it can be sterilized by autoclaving, according the classical method used for sterilization of pure bicarbonate solution i.e. at 100°C for one hour, included in air tight plastic bag.

Sterilized PD solution protected from light and stored at temperature varying from 10 to 35°C, remained stable for at least six months. No precipitate was observed. All components contained into the single bicarbonate amino acid-based solution remained practicaly unchanged as the difference between monthly measurements did not exceed ±1.3%. The pH of the solution, ranged withing its initial values (7.25 -7.45). On the other hand, the non sterilized solution of HD, even exposed on air preserves its initial pH (7.25-7.45) for more than 8 hr. Apparent formation of insoluble neutral calcium and magnesium carbonate were not observed. Bicarbonate dialysate prepared on-line is rapidly deteriorated during the HD session, and the insoluble calcium and magnesium carbonate deposition reappear on the wall of flow path tubes.

### Comparative study of single amino acid- bicarbonate solution vs lactate solution with 2.5% glucose in rabbits for assessing ultrafiltration

Two groups of six rabbits (2.5-3.0 Kg) each received i.p. 40 mL/ Kg of either single bicarbonate amino acid-based solution (osmolarity 369 mOsm) and a currently used lactate solution, containing 25 g/L of glucose (osmolarity 408 mOsm), respectively. After a 6-hr dwell time, the animals were sacrificed and the peritoneal fluid was collected, according to a technique reported elsewhere (6). The fluid was measured and net ultrafiltration was calculated as the difference between the infused and drained volume. The mean value of net ultrafiltration was appoximately 25% greater in rabbits received single bicarbonate amino acid-based solution than that with lactate solution (59 ± 2.66 vs 48 ± 2.5, p< 0.05), although the osmolarity of the first solution was only 369 mOsm and the average mw of amino acids 154.2 against the second solution with an osmolity 408 mOsm and a mw of glucose 180. Apparently this increased ultrafiltration may be attributed to either the constant normal pH of the single bicarbonate amino acid-based solution, the presence of amino acids, the absence of glucose and its antioxidant property of PD single bicarbonate solution. Other factors may contribute to increased ultrafiltration. As amino acids accept and donate protons, that is, when they lose protons become negatively charged or when they gain protons become positively charged, it is possible in either of situations that absorption of given indispensable amino acids is repeled from the peritoneum. High ultrafiltration let us to expect that our PD solution would effectively treat the patients by 2 or 3 exchanges daily.

### Comparative study of single bicarbonate dialysate vs bicarbonate on-line dialysate in a volunteer patient for assessing various parameters

Single bicarbonate HD dialysate was used for a 3-month period in a volunteer patient undergoing regular chronic HD with bicarbonate dialysate prepared on-line for 3 yrs. Biochemical values were compared with those of the last 3- month period. Patient served as his own control. The patient tolerated the single bicarbonate dialysate well, as he did previously with the bicarbonate dialysate prepared on-line. There were no significant differences in the following parameters: urea, creatinine, hematocrite, hemoglobin, Na, K, P, Cl, Ca, Mg and PaCO₂ An insignifican increase of pH and HCO₃ values before and after HD with single HD dialysate was observed, while a reduction (p<0.05) of the oxidative activity of serum was noted. This could be attributed to the scavengering property of aspartic acid and glutamic acid, which may attenuate the rate of atheromatosis in chronic uremic patient.

### References

1. Yatzidis H: A new stable bicarbonate dialysis solution for peritoneal dialysis. Preliminary report. Perit Dial Int 1991; 11: 224-227.
2. Yatzidis H: A new single bicarbonate CAPD solution. In LaGreca G, Ronco G, Feriani M, Chiaramonte S, Conz P (Eds) Peritoneal Dialysis. Milano. Wichting Editore, 1991 pp 151-157.
3. Yatzidis H: Hemodialysis with a new single stable bicarbonate dialysate. Nephron 1993; 64: 27-31.
4. Yatzidis H: Enhanced ultrafiltration in rabbits with bicarbonate glycylglycine peritoneal dialysis solution. Perit Dial Int 1933; 13: 302-306.
5. Yatzidis H: Effect on the peritoneal membrane of rabbits of a single bicarbonate solution containing glycylglycine. Adv Perit Dial 1994; 10: 251-255.
6. Yatzidis H, Dombros NV, Digenis GE: On the usefulness of glycylglycine in hemodialysis and peritoneal dialysis solutions. ASAIO J 1996; 42: 984-992.
7. Slingeneyer A, Pryzbylski C, Rolland R, Mion C: A new bicarbonate solution for CAPD (Abstract). Perit Dial Int 1993: 13 (Suppl 1) : 57.
8. Slingeneyer A, Faller B, Michel C, Pryzbylski C, Rolland R, Mion C: Increased ultrafiltration capacity using a new bicarbonate CAPD solution (Abstract) Perit Dial Int 1993; (Suppl 1): 57.
9. Pedersen FB: Biocompatibility studies with bicarbonate -based solution. Adv Perit Dial 1994; 10: 245-250.
10. Fougeray S, Slingeneyer A, Bastide JM, Mion C: Dialysis solutions buffered with lactate or bicarbonate: in vitro comparison of two dialysis solutions on human peritoneal cell growth from ESRD et non-ESRD patients. Adv Perit Int 1994; 10: 235-240.
11. Fischer F-P, Schenk U, Kiefer T, Hubel E, Thomas S, Yatzidis H, Mettang T, Kuhlmann U: In vitro effects of bicarbonate versus lactate buffered CAPD fluid on PMO function. Am J Kidney Dis 1995; 24: 924-933.
12. Yatzidis H: New single bicarbonate/ L-aspartic acid/ L-glutamic acid/ glucose (Bi/AAGA/GLU) solutions of physiological pH and variable osmotic pressure for peritoneal dialysis ( May 18, 1999 Number of patents 1003136, 1003134 and 1003133).
13. Dudrick SJ, Wilmore DW, Vars HM, Rhoads JE: Long-term total parenteral nutrition with growth, development, and positive nitrogen balance. Surgery 1968; 64: 134-142.
14. Gjessing J: Addition of amino acids to peritoneal dialysis fluid: Lancet 1968; 2: 812.
15. Oreopoulos DG, Crassweller P, Katirtzoglou A, Ogilvie R, Zellerman G, Rodella H, Vas SI. Amino acids as an osmotic agent (instead of glucose) in continuous ambulatory peritoneal dialysis; in Legrain M (Ed) CAPD. London. Oxford- Princetown 1980, pp 335-340.
16. Osborne TB, Mandel LB: Amino acids in nutritionand grawth. J Biol Chem 1914; 17, 325.
17. Rose WC: The amino acid requirement of adult man. Nutr Abstr Rev 1957; 27: 631-47.
18. Irwin MI, Hegsted DM: A conspectus of research on amino acid requirement of man. J Nutr 1971; 101: 539-566.
19. Laidlaw SA, Kopple JD: Newer concepts on the indispensable amino acids. Am J Clin Nutr: 1987; 46:593-605.
20. The Merck Index: Twelfth Edition 1996, MERCK & C.O., INC, Whitehouse Station, NJ, pp 132.
21. Fishman WH, Artom C: Serine injury. J Biol Chem 1942; 145: 345-349.
22. Nasset ES, Gatewood, VH: Nitrogen balance and hemoglobin of adult rats fed amino acid diets low in L-and D-histidine. J Nutr 1956; 53: 163-176.
23. Stifel FB, Herman RH: Is histidine an essential amino acid in man? Am J Clin Nutr 1972; 25:182-185.
24. Kopple JD, Swendseid ME: Evidence that histidine is an essential amino acid in normal and chronically uremic man. J Clin Invest 1975; 55:881-891.
25. Kopple JD, Swendseid ME: Effect of histidine intake on plasma and urine histidine levels, nitrogen balance and N-methyl histidine excretion in normal and chronically uremic men. J Nutrl 1981; 111: 931-942.
26. Terry BE, Yamanaka WK, Anderson HL, Wixom RL: Total parenteral nutrition with selective histidine depletion in man. II Hematological, lipid and ormonal responces. Am J Clin Nutr 1977; 30: 900-909.
27. Cho ES, Anderson HL, Wixom RL, Hanson KC, Krause GF: Long -term effects of low histidine intake on men. J Nutr 1984; 114: 369-384.
28. Mercer LP, Dodds SJ, Weler MD, Dum JD. Histidine histamine, and neuroregulation of food intake: a review and hypothesis. Nutrition 1990; 6:273-277.
29. Giordano C, De Santo NG, Rinaldi S, Pascale C, Pluvio M: Histidine and glycine essential amino acids in uremia. In: Kluthe R, Berlyne G, Burton B (eds). Uremia, an international conference on pathogenesis, diagnosis and therapy. Stuttgart: George Thiene Verlag, KG 1972; 138-143.
30. Irwing MI, Hegsted DM: A conspectus of research on amino acid requirements of man. J Nutr 1971; 101: 539-566.
31. Fellows FCI, Lewis MHR: Lysine metabolism in mammals. Biochem J 1973; 136:329-334.
32. Goulet O, DePotter S, Salas J, Robert J-J, Rongier M, Hariz MB, Koziet J, Desjeux J-F, Rigour C, Darmaun D: Leucine metabolism at graded amino acid intakes in children receiving parenteral nutrition. Am J Physiol 1993; 265: (Endocrinol Metab, 28): E 540-E 546.
33. Ballevre O, Prugnaud J, Houlier ML, Arnal M: Assessment of threonine metabolism in vivo by gas chromatography/mass spectrometry and stable isotope infusion. Anal Biochem 1991; 193: 212-219.
34. Kamoun P: Valine is a precursor of propionyl-CoA. TIBS 17 may 1992 (Textbook Errors) pp 175-176.
35. Young SN: Use of tryptophan in combination with other antidepressant treatment: A review. J Psychiatry Neurosci 1991; 16: 241-269.
36. Batshaw ML, Valle D, Bessman SP: Unsuccessful treatment of phenylketonuria with tyrosine. J Pediat 1981; 99: 159-160.
37. Wurtman RJ: Nutrients that modify brain function. Sci Am 1982; 246: 50-59
38. Reyes AA, Karl IE, Klahr S: Role of arginine in healt and in renal diseases. Am J Physiol 1994; 267 (Renal Fluid Electrolyte Physiol 36): F 331-346.
39. Tomé LA, Yu L, deCastro I, Campos SB, Seguro AC: Beneficial and harmful effects of L-arginine on renal ischaemia, Nephrol Dial Transplant, 1999; 14: 1139-1145.
40. Rossitch E, Alexander E, Black PM, Cooke JP: L-arginine normalizes endothelium function in cerebral vessels from hypercholesteremic rabbits. J Clin Invest 1991; 87: 1295-1299.
41. Cooke JP, Singer AH, Tsao FS, Zera P, Rowan RA, Billingham E: Antatherogenic effects of L-arginine in the hypercholesterolemic rabbit. J Clin Invest 1992; 90: 1168-1172.
42. Tsao PS, McEvoy LM, Drexler H, Butcher EC, Cooke JP: Enhanced endothelial adhesiveness in attenuated by L-arginine, Circulation 1994; 89:2176-2182.
43. Böger RH, Bode-Böger SM, Mügge A, Kien Ke S, Brandes R, Dwenger A, Frölich JC: Supplementation of hypercholesterolaemic rabbits with L-arginine reduces the vascular release of superoxide anions and restores NO production. Atherosclerosis 1995; 117:273-284.
44. Böger RH, Bode-Böger SM, Kienke S, Stan AC, Nafe R, Frölich JC: Dietary L-arginine decreases myointimal cell proliferation and vascular monocyte accumulation in cholesterol-fed rabbits. Atherosclerosis 1998; 136: 67-77.
45. Bode-Böger SM, Böger RH, Kienke S, Böhme M, Phivthog-ngam L, Tsicas D, Frölich JC: Chronic dietary supplementation with L-arginine inhibits platelet aggregation and thromboxane A₂ synthesis in hypercholesterolaemic rabbits in vivo. Cardiovascular Resears 1998; 37: 756-764.
46. Drexler H, Zeiher AM, Meinzer K, Just H: Correction of endothelial dysfunction in coronary microcirculation of hypercholesterolaemic patients by L-arginine. Lancet 1991; 338: 1546-1550.
47. Cooke JP, Dzau J, Creager A: Endothelium disfunction in hypercholesterolemia is corrected by L-arginine Basic Res Cardiol 1991; 86 (Suppl 2): 173-181.
48. Clarkson P, Adams MR, Powe AJ, Donald AE, McCredie, Robinson J, McCarthy SN, Keech A, Calermajer DS, Deanfield JE: Oral L-arginine improves endothelium -dependent dilation in hypercholesterolemic young adult. J Clin Invest 1996; 97: 1989-1994.
49. Moncada S, Palmer RM, Higgs EA: Nitric oxide: physiology, pathophysiology and pharmacology. Pharmacol Rew 1991; 43: 109-142.
50. Schramm L, Heidbreder E, Schmitt A: Role of L-arginine -derived NO in ischemic acute renal failure in the rat. Renal Failure 1994; 16: 555-569.
51. Agmon Y, Peleg H, Greenfeld, Rosen S, Brezis M: Nitric oxide and prostanoids protect the real ourer medulla from radiocontrast toxicity in the rat. J Clin Invest 1994; 94: 1069-1075.
52. Andrade L, Campos SV, SeGuro AC: Hypercholesterolemia aggravates radiocontrast nephrotoxicity: Protective role of L-arginine. Kidney Int 1998; 53: 1736-1742.
53. Yu L, Gengaro PE, Niederberger M, Burke TJ, Schrier RW: Nitric oxide a mediator in rat tubular hypoxia / reoxygenation injury. Proc Natl Acad Sci USA 1994; 91: 1691-1695.
54. Bulus N, Cersomimo E, Chisham F, Abumrad NN: Physiology importance of glutamine. Metabolism 1982; 38 (Suppl 1): 1-5
55. Souba WW, Klimberg VS, Plumley DA, Salloum RM, Flyn TC, Bland KI, Copelana EM: The role of glutamine in maintaining a healthy gut and sopporting the metabolic responce to injury and infection. J Surg Res 1990; 98: 383-391.
56. Smith R: Glutamine metabolism and its physiologic importance. IPEN 1990; 14: 405-445.
57. Perriello G, Jorde R, Nurjhan N, Stumvoll M, Dailly G, Jenssen T, Bier DM, Gerich JE: Estimation of the glucose-alanine-lactate-glutamine cycles in postabsorptive man: Role of sceletal muscle. Am J Physiol 1955; 269: E 443- E 450.
58. Stumvoll M, Perriello G, Meyer C, Gerich J: Role of glutamine in human carboxydrate metabolism in Kidney and othe tissues.Kidney Int 1999; 55: 778-792.
59. Nurghan N, Bucci A, Perriello G, Stumvoll M, Dailey S, Bier D, Toft I, Jenssen T, Gerich J: Glutamine : A major gluconeogenic precursor and vehicle for interorgan carbon transort in man. J Clin Invest 1995; 95: 272-277.
60. Hankard RG, Darmaun D, Sager BK, D' Amore D, Parsons WR, Haymond M: Responce of glutamine metabolism to exogenous glutamine in humans. Am J Physiol 1995; 269: E 663-E 670
61. Varnier M, Leese GP, Thompson J, Rennie MJ: Stimulatory effect of glutamine on glycogen accumulation in human skeletal muscle. Am J Physiol 1995; 269: E 309- E 315.
62. Kreider M, Stumvoll M, Meyer C, Overkamp D, Welle S, Gerich J: Steady state and non-steedy measurement of plasma glutamine turnover in human. Am J Physiol 1997; 272: E 621- E 627
63. Perriello G, Nurihan N, Stumvoll M, Bucci A, Welle S, Dailey G, Bier DM, Tolf I, Jenssen TG, Gerich JE: Regulation of gluconeogenesis by glutamin in normal postbsorptive human. Am J Physiol 1997; 272: E 437- E 445.
64. Stumvoll M, Meyer C, Kreider M, Perriello G, Gerich J: Effects of glucagon on renal and hepatic glutamine gluconeogenesis in normal postabsorptive human. Metabolism 1998; 47: 1227-1232.
65. Lavoinne A, Baquet A, Hue L: Stinulation of glucogen synthesis and lipogenesis by glutamine in isolated rat hepatocytes. Biochem J 1987; 248: 429-437.
66. Cercosimo E, Williams P, Hoxworth B, Lacy W, Abumrad N: Glutamine blocks lipolysis and ketogenesis of fasting. Am J Physiol 1986; 250: E 248-252.
67. Wu G, Thompson JR: The effect of glutamine on protein turnver in chick skeletal muscle in vitro. Biochem J 1990: 265: 593-598.
68. Pösö A, Schworer C, Mortimore G: Acceleration of proteolysis in perfused rat liver by deletion of glucogenic amino acids: Regulatory role of glutamine. Biochem Biophys Res Commun 1982; 107: 1433-1439.
69. Seglen PO, Gordon PB, Poli A: Amino acid inhibition of the autophagic /lysosomal pathway of protein degradation is isolated rat hepatocytes. Biochem Biophys Acta 1980; 630: 103-118.
70. Mac Lennan P, Smith K, Weryk B, Watt P, Rennie M: Inhibition of protein breakdown by glutamine in perfused rat sceletal muscle. FEBS Lett 1988: 133-136.
71. Jepson M, Bates P, Broadbent P, Peli J, Millward D: Relationship between glutamine concentration and protein synthesis in rat skeletal muscle. Am J Physiol 1988; 255: E 166- E 172.
72. Ismail N, Brouillette JR , Mujais SK : Hemodialysis Technology. In: Clinical Nephrology, Dialysis and Transplantation (Eds Malluche HH, Sawava BP, Hakim RM, Sayegh MH 1999 Dustriverlag Dr Karl Feistle, München pp 1-37.

## Claims

1. The newer proposed two single and stable solutions for peritoneal dialysis (PD) and hemodialysis (HD) contain the needed electrolytes, the bicarbonate and at very small concentration (1.5 mM/L) disodium hydrogen citrate. Besides, the PD solution contains the following ten indispansable amino acids, L-istidine, L-leucine, L-lysine, L-methionine, L-phenylalanine, L-threonine, L-tryptophane, L-valine, L-arginine and L-gloutamine, while the HD solution contains the two dispansable amino acids, L-aspartic acid and L-glutamic acid.

2. Both, PD and HD solutions has the same stable pH 7.25-7.45 arising in PD solution from the ten indispansable amino acids and bicarbonate, and in HD solution from the two dispansable amino acids and bicarbonate. These render PD and HD solution authentic buffers. Studies in our laboratory proved that at pH 7.25-7.45, citrate even at the very small concentration of 1.5 mL/L form by its chelating capability with calcium and magnesium stable soluble complexes, excluding so the appearance of neutral insoluble calcium and magnesium carbonate salts.

3. The soluble complexes of PD and HD solutions during their use are tranfered in the circulation of patient where citrate rapidly metabolized in liver to bicarbonate, and the released calcium and magnesium, regain their pure forms. Except the role of amino acid in the formation of pH 7.25-7.45 mentioned in claim 2, the ten indispansable amino acids of PD solution improve disturbed nutrition of patients, adjust the removal of water balance via the peritoneum, and as L-glutamine is potent scavenger, neutralizes the harmful reactive oxygen species (ROS). These exist in excess, causing the damage of peritoneum and all cells and tissues. Also, L-aspartic acid and L-glutamic acid, potent scavengers according our studies, neutralize ROS in patient undergoing hemodialysis by using HD solution.

4. PD and HD solutions are prepared by dissolving chemecals in freshly produced ultrapure water by combining, carbon perfusion, reverse osmosis and membrane filtration for retaining bacterials. PD dialysis solution could be sterilized by filtration, but also by autoclaving, using the classical method for sterilization of pure bicarbonate solutions at 100° C for one hour in air tight plastic bac. The sterilized PD solution protected from light and stored at a temperature varying from 10° to 35° C remains stable for at least six months.

5. HD solution preparation is performed by dissolving a hundredfold dry mixture of of all needed chemicals (984.5 g) at 100 L of freshly ultrapure water made hand or mechanicaly, just before HD session,

6. HD solution, even exposed in air preserves its initial pH (7.25-7.45) for more than 8 hr after its preparation. During this time formation of insoluble neutral calcium and magnesium carbonate salts never were observed. On the contrary bicarbonate solution prepared on-line is rapidly deteriorated during the HD session with visible depositions on the inside wall of flow path tubes.

7. PD solution vs standard lactate solution with 25 g/L glucose in rabbits, and HD solution vs on-line prepared HD bicarbonate solution in a human volunteer, compared as above, proved the significant superiority of newer solutions in terms to ultrafiltration, pH, acid -base control and oxidative activity.
